**Europäisches Patentamt**

**European Patent Office**

**Office européen des brevets**

(11) Publication number : **0 472 286 A1**

# EUROPEAN PATENT APPLICATION

(21) Application number : **91306542.1**

(22) Date of filing : **18.07.91**

(51) Int. Cl.$^5$ : **C12N 15/55, C12N 9/14**

(30) Priority : **18.07.90 US 555123**

(43) Date of publication of application :
**26.02.92 Bulletin 92/09**

(84) Designated Contracting States :
**CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Kurtz, Myra B.**
**16 Redwood Road**
**Martinsville, NJ 08836 (US)**
Inventor : **Marrinan, Jean A.**
**74 Avebury Place**
**Somerset, NJ 08873 (US)**

(74) Representative : **Thompson, John Dr. et al**
**Merck & Co., Inc. European Patent**
**Department Terlings Park Eastwick Road**
**Harlow, Essex CM20 2QR (GB)**

(54) Gene encoding candida albicans plasma membrane H+ATPase.

(57) A unique gene encoding the plasma membrane H+ATPase of the fungi Candida albicans has been isolated, purified, cloned and sequenced. The cloned gene is free of other Candida nucleic acids and is used to produce Candida albicans plasma membrane H+ATPase. The unique Candida albicans plasma membrane gene can be used to transform non-pathogenic yeast which can be used to evaluate agents capable of perturbing Candida albicans plasma membrane H+ATPase activity.

EP 0 472 286 A1

FIG. 1

## BRIEF DESCRIPTION OF THE DRAWINGS

FIGURE 1. Restriction map depicting the location of sequences recognized by restriction enzymes capable of cleaving the DNA encoding Candida albicans plasma membrane H+ATPase.

FIGURE 2A-H. Nucleotide and amino acid sequence for Candida albicans plasma membrane H+ATPase including the location of endonuclease restriction sites.

## BACKGROUND OF THE INVENTION

The plasma membrane H+ATPase of fungi and plants perform an essential role in these organisms by maintaining internal pH and membrane potential required for active transport of nutrients. The proton gradient generated by the enzyme results in the active transport of nutrients by H+-symport and may be involved with pH balances that mediate cell growth, Serrano et al., Nature 319: 689-693 (1986). The genes encoding fungal H+-ATPase (PMA1 and PMA2) have been isolated, cloned and sequenced from Saccharomyces cerevisiae (Sarrano et al., Nature 319: 689-693 [1986]) and (Schlesser et al., J. Biol. Chem. 263: 19480-19487 [1988]), Neurospora crassa (Hager et al., Proc. Natl. Acad. Sci. U.S.A. 83: 7693-7697 [1986]) and Schizosaccharomyces pombe (Ghislaim et al., J. Biol. Chem. 262: 17549-17555 [1987]). This class of enzymes has no direct counterpart in mammalian cells (Serrano, R. Plasma Membrane ATPase of Plants and Fungi, CRC Press, 1985). The present invention suggests that this enzyme can function as a possible target for differential drug therapy for fungal disease. The gene encoding the H+ATPase from Candida albicans has been cloned and sequenced, see below. Therefore the enzyme form a pathogenic fungus can be produced in excess, genetically manipulated and studied in detail as an antifungal target.

## OBJECTS OF THE INVENTION

It is, accordingly, an object of the present invention to isolate and purify a gene capable of encoding Candida albicans plasma membrane H+ATPase. Another object is to provide for expression of the plasma membrane H+ATPase gene. A further object is to prepare organisms transformed with the Candida albicans plasma membrane H+ATPase gene.

## SUMMARY OF THE INVENTION

A unique gene encoding the plasma membrane H+ATPase of the fungi Candida albicans has been isolated, purified, cloned and sequenced. The cloned gene is free of other Candida albicans nucleic acids and is used to produce Candida albicans H+ATPase. The unique Candida albicans plasma membrane gene can be used to transform non-pathogenic yeast which can be used to evaluate agents capable of perturbing Candida albicans plasma membrane H+ATPase activity.

## DETAILED DESCRIPTION OF INVENTION

The present invention relates to the isolation, characterization, sequencing and expression of the Candida albicans plasma membrane H+ATPase gene. This will allow the enzyme from this pathogenic fungus to be produced in excess, genetically manipulated and studied in detail as an antifungal target. The plasma membrane H+ATPase gene is obtained from the DNA of the dimorphic pathogenic fungus Candida albicans, which is capable of producing the enzyme. Strains or species of such fungi include, but are not limited to strains known in the art and readily available from the American Type Culture Collection: Candida albicans ATCC 10261, ATCC 44990 and ATCC 22114.

Chromosomal DNA is isolated from pure cultures of microorganisms known to produce the ATPase, preferably Candida albicans ATCC 10261, by methods known in the art, as illustrated by Sherman, et al., (1983, Methods in Yeast Genetics, *Cold Spring Harbor Laboratory Press*, Cold Spring Harbor, N.Y.). The chromosomal DNA is fragmented, for example, by digestion, partially or completely with one or more restriction endonuclease enzymes, such as BamHI, ClaI, BclI, BglII, KpnI, Sau3A, or XhoI, with Sau3A being preferred. The digested DNA fragments are separated by size, and the size-specific fragments, about 2 to about 15 kb in length, are inserted into a cloning vector. Cloning vectors as used herein is defined as a DNA sequence which allows the incorporation of specific experimental foreign DNA, with the combined DNA being introduced into a host cell that can exist in a stable manner and express the protein dictated by the experimental DNA. The foreign DNA combined with the vector DNA constitutes a recombinant DNA molecule which is derived from recombinant technology. Cloning vectors may include plasmids, bacteriophage, viruses, and cosmids. It is to be understood that any cloning vector may be used to clone novel C. albicans DNA sequences, with plasmids being preferred. The cloning vector is cut with a restriction endonucleases such as BamHI, treated with phosphatase and the DNA fragments are ligated with a DNA ligase, T4 DNA ligase is preferred. The cloning vectors are used to transform host cells competent for the uptake of DNA. Competent host cells which may be used for cloning, DNA processing and expression generally include bacteria, yeast, fungi, insect, plant or mammalian cells with the preferred hosts being bacteria and yeasts. The most preferred yeasts being from the genera Candida and Saccharomyces and the most preferred bacteria being from the genus Escherichia. The most preferred Escherichia hosts include E. coli K-12 strains RR1, HB101, JM109 or DH597α. It has been determined herein that libraries consisting of about $5 \times 10^4$ independent inserts are likely to contain a representation of the entire genome. Examples of such libraries are described in Kurtz et al.,Genetic of Candida, CRC Press (1990), Magee, Anal. Biochem.175: 361-372 (1988), Gillum et al., Mol.Gen. Genet.198: 179-182 (1986), and Rosenbluh et al., Mol. Gen. Genet. 200: 500-502 (1985). Since the H⁺ATPase from yeast, fungi and plants are highly conserved, the C. albicans libraries are screened for clones which are homologous to the conserved sequences of the H⁺-ATPase genes of these organisms. Published DNA sequences from organisms which can be used include, but are not limited to: S. cerevisiae, (Serrano et al., Nature 319: 689-693 [1986], Schlesser et al., J. Biol. Chem. 263: 19480-87 [1988]); Neurospora crassa (Addison, J. Biol. Chem. 261: 14896-901[1986], Hager et al., Proc. Natl. Acad. Sci. USA 83: 7693-7697 [1986]), Schizosaccharomyces pombe (Ghislain et al., J. Biol. Chem. 262: 17549-55 [1987]), and Arabidopsis thaliana (Harper et al., Proc. Natl. Acad. Sci. USA: 86: 1234-38 [1989], Pardo and Serrano, J. Biol. Chem. 264: 8557-8562 [1989]) with the S. cerevisiae sequence being preferred. The libraries are screened with labelled fragments of the cloned genes or oligonucleotides generated from the published sequences prepared by standard procedures. Labelled probes can be prepared with radioisotopes, with ³²P being preferred, or with nonisotopic methods such as biotinylation. All processes are well known in the art.

The host cells containing cloned C. albicans DNA which shows homology with S. cerevisiae PMA1 DNA are propagated and the DNA extracted by routine methods known in the art. The gene encoding C. albicans plasma membrane H⁺ATPase (PMA 1), carried on about a 12 kilobase (kb) insert, is transfered to one or more vectors designed for the production of double-stranded DNA (dsDNA) which are useful for sequence analysis and for restriction mapping. Such vectors include, but are not limited to, pBR322, YEp13, and YEp24 (available commercially), and pGEM3zf(+) and pGEM5zf(+) (Promega). Restriction mapping of the dsDNA is performed with a variety of commercially available restriction enzymes by procedures known to those stilled in the art and the results are compared to genomic restriction maps of Candida albicans DNA. The genomic map is constructed by extracting chromosomal DNA from Candida albicans which is capable of producing the enzyme. Such fungi include, but are not limited to strains known in the art and readily available: Candida albicans ATCC 10261, ATCC 22114 and ATCC 44990. Chromosomal DNA is extracted by methods well know to the art (Sherman, F., et al., 1983, Methods in Yeast Genetics, Cold Spring Harbor Laboratory Press, Cold Spring Harbor, N.Y.). A portion of the chromosomal DNA is cut to completion with a number of restriction enzymes, such as EcoRI, HindIII, EcoRV, ClaI, and XhoI. As required, some DNA fragments are digested with more than one enzyme. The digested fragments of DNA are separated by gel electrophoresis. The fragments are then transferred to a solid membrane support such as nitrocellulose or nylon membrane with nylon membrane being the preferred support. The nylon blot is then hybridized with a radiolabelled probe. The radioisotope of choice is ³²P. The DNA fragment is radiolabeled either by a nick translation procedure (Rigby et al., J. Mol. Biol. 113: 237-251 [1977]) or a random priming procedure (Feinberg and Vogelstein, Anal. Biochem. 132: 613 [1983]), with the random priming procedure being the technique of choice. The hybridization blot is incubated overnight with the homologous radiolabeled fragment, with the about 2 kb KpnI from the S. cerevisiae PMA1 gene as the preferred probe (Serrano et al., Nature 319: 689-693 [1986]). The following day the blot is washed by methods known to the art (Maniatis et al.,1982, Molecular Cloning: A Laboratory Manual, Cold Spring Harbor Laboratory Press, Cold Spring Harbor). The blot is then exposed to XAR-5 film and developed by conventional methods (Laskey and Mills, FEBS Letters, 82: 314-316 [1977]). The size and pattern of the digested fragments which hybridize with the probe generate a genomic map. Comparisons of the genomic map with the restriction map of the cloned sequence confirm the identity of the cloned gene with the desired genomic sequence.

Since the Candida albicans plasma membrane H⁺ATPase gene is cloned by homology to sections of the Saccharomyces cerevisiae PMA1 gene, their DNA sequences should have a certain degree of homology to each other and the Saccharomyces sequence should act as a guide for sequencing the Candida DNA. The area of highest homology in the Candida clone is in the about 2.0 kb EcoRV fragment, and that is the segment preferred for initial sequencing. This fragment is subcloned into vectors which allow elucidation of the base sequence of the insert, such as pGEM5zf(+) which is a plasmid commercially available from Promega. Double stranded DNA is prepared from each of the insertion plasmids by the method Lim and Pene, Gene Anal Techn 5: 32-39 (1988). A double stranded sequencing procedure known in the art is used to determine sequence

4

data(Lim and Pene, Gene Anal. Tech. 5: 32-39 [1988] and Sequenase kit protocol manufactured by US Biochemical Corp.). Various primers can be used for sequencing, with the commercially available T7 and SP6 primers which are adjacent upstream and downstream, respectively, to the 2.0 kb EcoRV Candida fragment in this vector being preferred. Regions not accessible with the primers were sequenced by use of synthetic oligonucleotides primers.

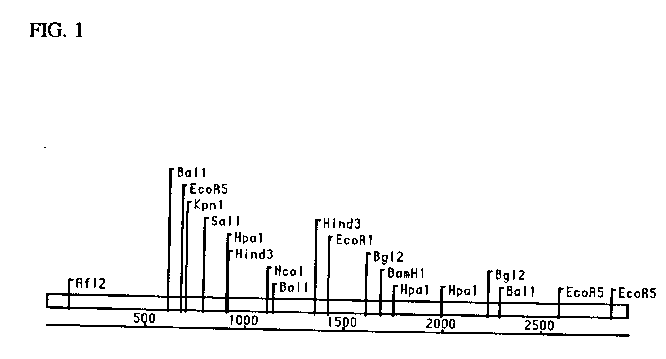

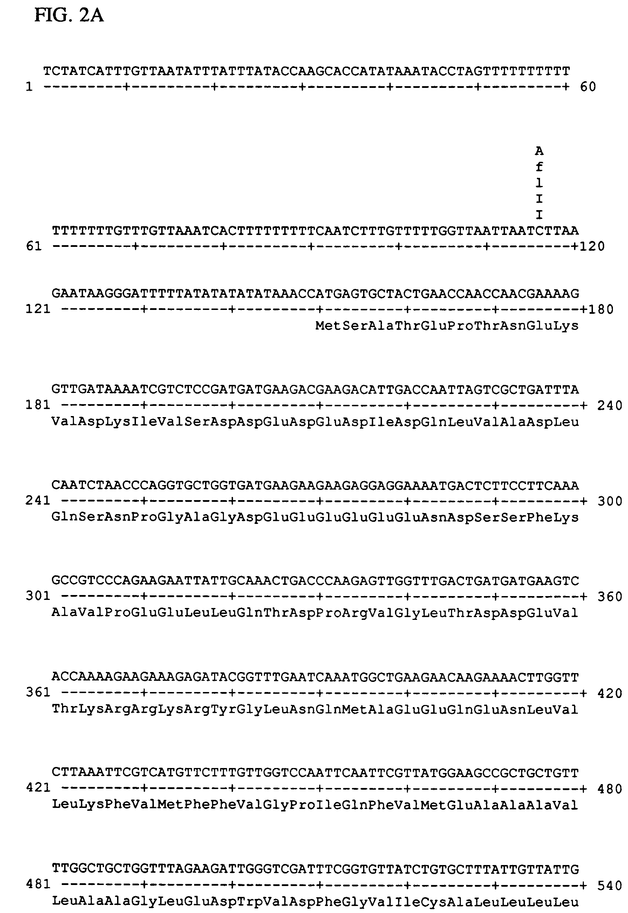

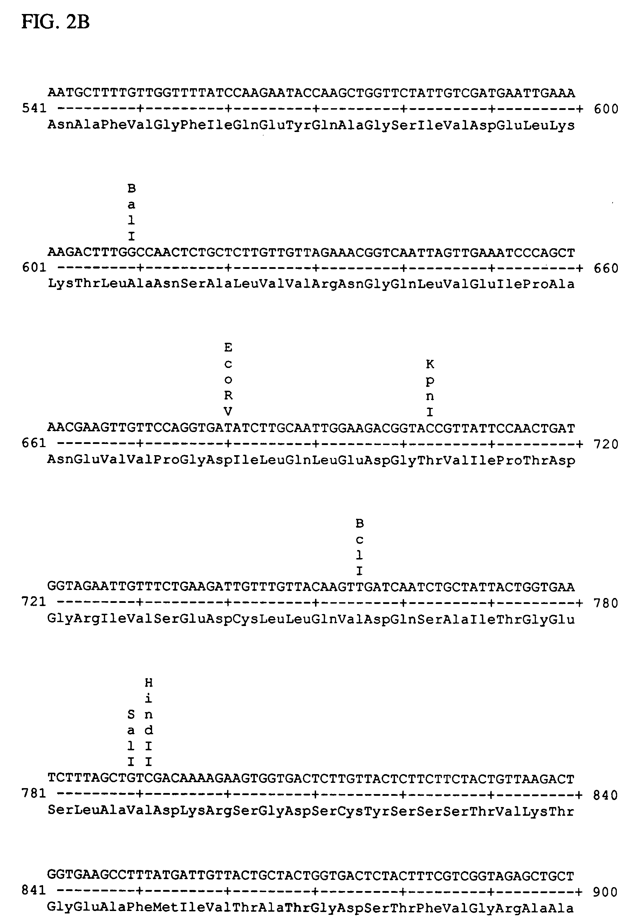

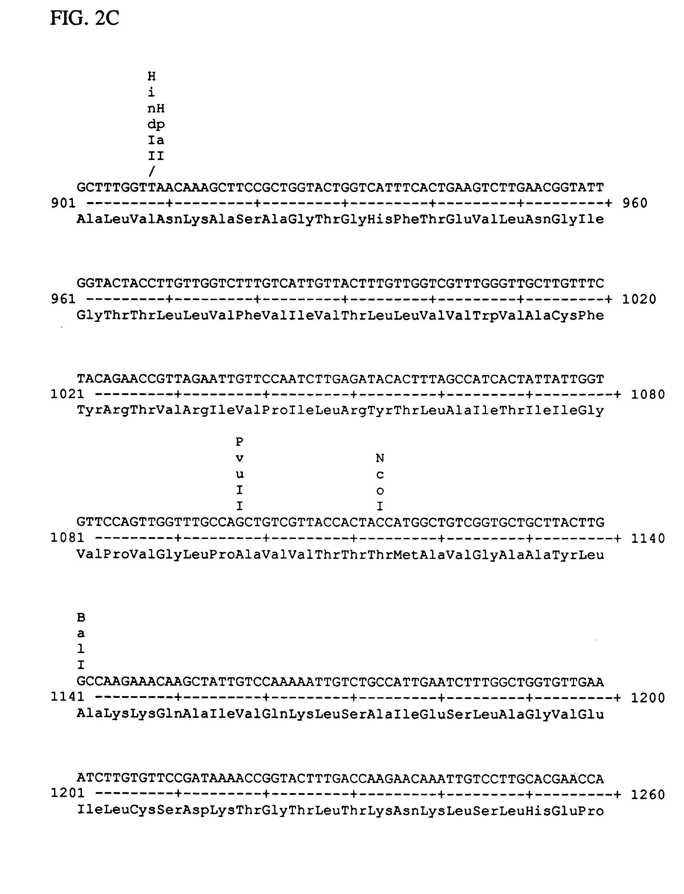

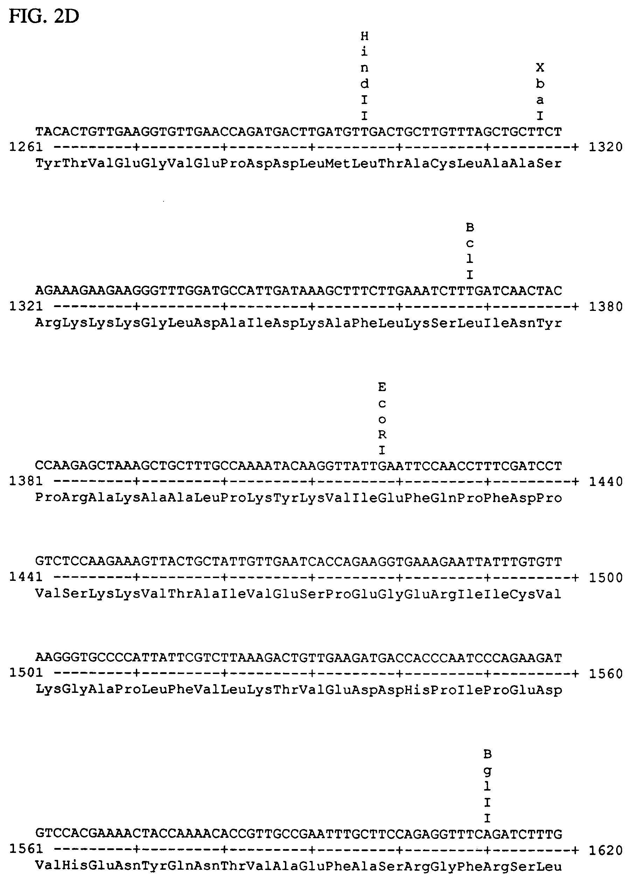

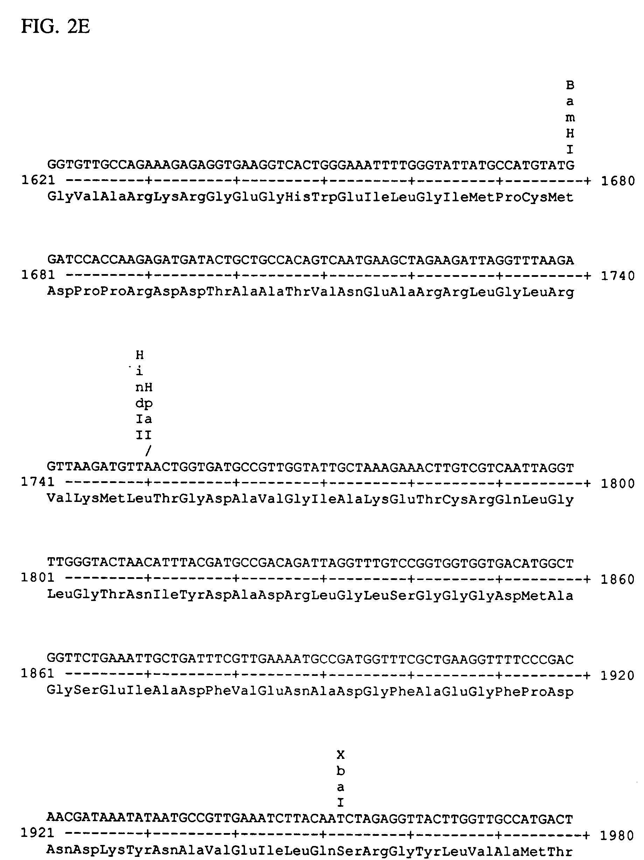

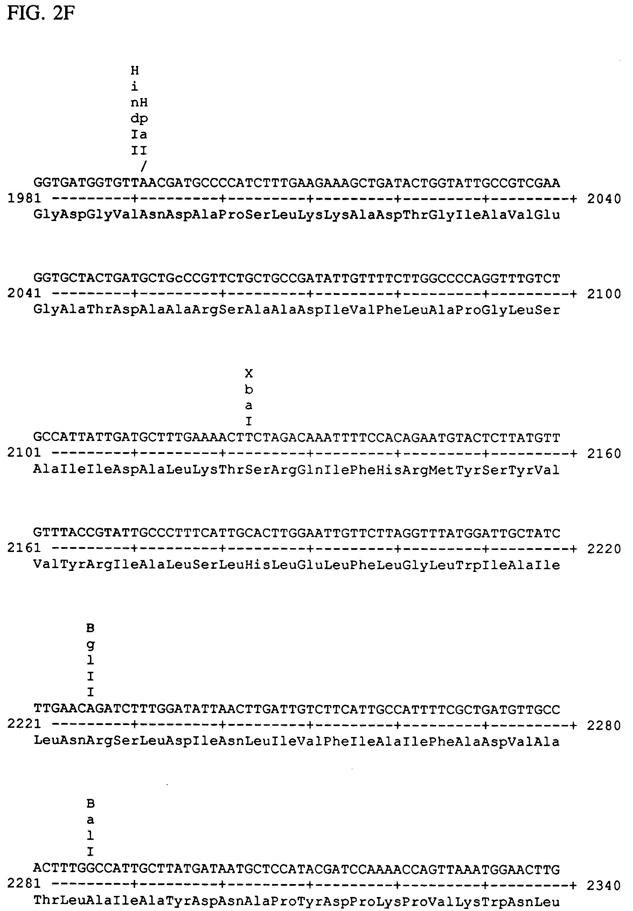

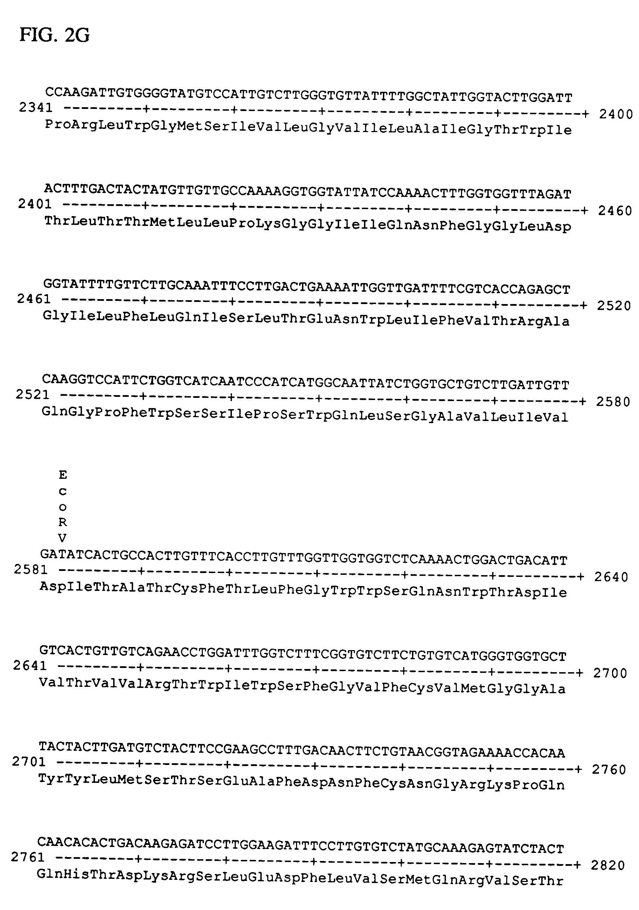

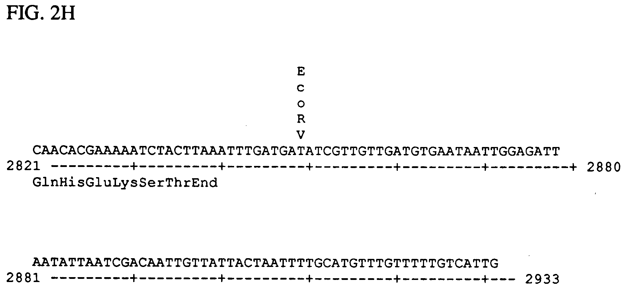

The DNA sequence of the cloned Candida albicans plasma membrane H$^+$ATPase is presented in Figure 2. The overall length of the cloned C. albicans DNA is about 2.7 kilobase pairs and is shown in Fig. 2. The cleavage sites for selected restriction endonucleases are indicated above the sequence and are shown in Fig. 1. The predicted amino acid sequences for the C. albicans Plasma membrane H$^+$ATPase gene are shown below the DNA sequence in Fig. 2. Analysis of the N terminal region of the DNA sequence located an open reading frame that starts at nucleotide about 151. Analysis of about the first 897 amino acids indicates that this peptide is similar to the Saccharomyces cerevisiae PMA1 gene: about 84% identity in about a 794 amino acid overlap. This analysis also shows a high degree of similarity between three other fungal PMA genes: S. cerevisiae PMA2, about 84% identity in about a 847 amino acid overlap; S. pombe PMA1 , about 75% identity in about a 794 amino acid overlap; and N. crassa PMA1 gene, about 76% identity in about a 890 amino acid overlap. It is intended that the nucleotide sequence for C. albicans H$^+$ATPase be interpreted to include all codons that code for the approprioto amino acid in the sequence for H$^+$ATPase, as indicated by the degeneracy of the genetic code. It is also intended that the invention include all normal mutations of the H$^+$ATPase base sequence that may result in a biologically active enzyme. It is further intended that the nucleotide sequence for the ATPase include truncated genes which encode biologically active fragments of the ATPase. It is intended that the pharse altered forms refer to the above changed in the Candida albicans H$^+$ATPase gene. Biologically active as used herein includes the ability of the recombinant gene product to exhibit plasma membrane H$^+$ATPase activity.

The purified gene is incorporated into an appropriate vector and used to transform a host cell capable of supporting expression of the gene product. Host cells include, but are not limited to, bacteria, fungi, blue green algae, plant cells and animal cells. The host cells include bacteria and fungi, with the preferred host cells being fungi. The most preferred fungal cells include, but are not limited to those in the genera Saccharomyces and Candida. Cloning of the Candida albicans gene into Saccharomyces cerevisiae and Candida albicans is most easily accomplished by subcloning specific Candida DNA fragments into appropriate vectors which allow expression of the Candida gene from its own promoter or from promoters on the cloning vector. A promoter is defined as a DNA sequence that is upstream of the experimental gene, C. albicans H+ATPase, that directs RNA polymerase to bind to DNA and to initiate RNA synthesis resulting in mRNA production from the experimental gene. A strong promoter is one which causes mRNAs to be initiated at high frequency. The about 12 kb ClaI Candida albicans Plasma membrane ATPase fragment or smaller fragments which contain the entire coding region is subcloned into a plasmid such as pUN100 (Elledge and Davis, Gene 70: 303-312, 1988) containing a selectable marker for Saccharomyces such as the LEU2 gene and a CEN/ARS which enables it to replicate as a low copy plasmid in Saccharomyces cerevisiae. The about 12 kb ClaI fragment is obtained from a plasmid containing it, as described above, by enzymatic digestion with the restriction enzyme ClaI using standard techniques. The Candida gene is separated from vector components by standard gel electrophoresis techniques and purified by methods outlined in Maniatis et al., Molecular Cloning: A Laboratory Manual, Cold Spring Harbor Laboratory Press, Cold Spring Harbor (1982). The ClaI fragment is treated with T4 DNA to generate blunt ends. The recipient plasmid, pUN100, is cut with HindIII and also treated with T4 DNA polymerase to generate blunt ends. The ClaI/blunt fragment is ligated to the pUN100 using T4 DNA ligase. A similar insertion is constructed in YEp13 or similar vectors, which are plasmids containing a selectable marker for Saccharomyces such as the LEU2 gene and 2μ sequences which enable it to achieve a high copy number in Saccharomyces. The blunt ClaI fragment is inserted into a T4 DNA polymerase treated HindIII site on YEp13. The resulting plasmids are transformed into Sacchoromyces cerevisiae, strains such as RS-72 (Serrano et al., Current Genetics 12: 105-110 [1987] or other appropriately marked yeast strains. Transformation are performed by standard methods such as the spheroplasting method outlined by Sherman et al., Methods in Yeast Genetics, *Cold Spring Harbor Laboratory Press*, Cold Spring Harbor, N.Y. (1983).

Sequence analysis and restriction mapping of the Candida plasma membrane ATPase gene shows that the gene is fully contained on a 6 kb SnaBI fragment. pRC2312 is a plasmid derived from a plasmid described by Serrano et al., Mol. Gen. Genet. 221: 210-218 (1990). It has a Candida selectable marker, URA3, and has a Candida ARS which permits copies of 2-3 per genome. Other ARS vectors which are capable of producing high copy numbers in Candida are known in the art. The 6 kb SnaBI fragment is obtained from a plasmid containing it by enzymatic digestion with the restriction enzyme SnaBI using standard techniques. The fragment free of vector is obtained by standard gel electrophoresis techniques and purified by methods described above and in the examples. The fragment is subcloned into the SmaI site of pRC2312 using T4 DNA ligase. Since both restriction enzymes, SnaBI and SmaI, yield blunt ends, T4 DNA polymerase treatment is not needed. The resulting plasmid, designated pJAM30, is used to transform an appropriately marked strain such as Candida albicans strain 1006, Goshorn et al., Genetics 123: 667-673 (1989). The transformation is accomplished by the lithium acetate method of Kurtz, in Genetics of Candida, CRC Press, pp21-73 (1990).

The following examples illustrate the present invention without, however, limiting the same and modifications of the invention will be apparent to persons skilled in the art.

EXAMPLE 1

Cloning of the Candida albicans Plasma Membrane ATPase Gene

A single large colony of Candida albicans, ATCC 10261, was isolated from a culture plate containing YEPD (1% yeast extract, 1% peptone and 2% glucose) solidified with agar (1.5%) and used to inoculate 100 ml of YEPD broth. After incubation for 16 hours at 30°C, the cells were collected and the chromosomal DNA extracted by the method of Sherman, F., et al., Methods in yeast genetics, Cold Spring Harbor Laboratory Press, Cold Spring Harbor, N.Y. (1983). A portion of the chromosomal DNA (3 $\mu$g) was cut to completion with a number of restriction enzymes, such as EcoRI, HindIII, EcoRV, ClaI, and XhoI using conditions recommended by the manufacturer. The digested DNA fragments were separated by agarose gel electrophoresis to produce discrete bands whose size can be estimated by comparison with commercially available molecular size standards. The fragments were then transferred to GeneScreen nylon membrane (New England Nuclear) utilizing the process of Southern, J. Mol. Biol., 98: 503-517 (1975). The DNA fragments on the nylon blot were then hybridized with a $^{32}$P radiolabeled probe prepared by the random priming procedure of Feinberg and Vogelstein, Anal. Biochem. 132: 6-13 (1983).

The DNA fragment that was used to hybridize the Candida blot was a 2 kb KpnI fragment isolated from plasmid B1138 which contains the Saccharomyces cerevisiae Plasma membrane ATPase (PMA1) gene (Serrano et al., Nature, 319: 689-693 [1986]) in the pUC18 vector. Twenty micrograms of B1138 was digested with KpnI as recommended by the manufacturer. The digested plasmid was separated by electrophoresis on low gelling temperature agarose. The 2 kb band was excised from the gel and the DNA was extracted by procedures outlined by Maniatis et al., supra. The blot was hybridized overnight with the 2 kb KpnI radiolabeled fragment. The following day the blot was washed by methods described by Maniatis et al., Molecular Cloning: A Laboratory Manual, Cold Spring Harbor Laboratory Press, Cold Spring Harbor (1982). The blot was then exposed to XAR-5 film and developed by conventional methods (Laskey and Mills, FEBS Letters, 82: 314-316 [1977]). The probe hybridized to selected fragments of DNA which had homology to Saccharomyces DNA. When the Candida DNA was cut with EcoRI, 9.4 kb and 3.4 kb bands were generated. When HindIII was used, 7.1 kb and 3.6 kb bands were observed. With EcoRV, 5.0 and 1.9 kb bands are generated. Cleavage with ClaI resulted in a single 13 kb band. XhoI does not cut the Candida DNA. When the chromosomal DNA was digested both with HindIII and EcoRI, 3.6 and 3.4 kb fragments hybridize to the probe. With HindIII/EcoRV, 3.3 and 1.1 kb bands were generated. When EcoRI/EcoRV were used, 3.05, 1.0 and 0.7 kb bands were observed. From these observations a restriction map of the chromosomal DNA homologous to S. cerevisiae was generated. The blot illustrates that the Candida albicans DNA is homologous to the Saccharomyces cerevisiae 2kb KpnI fragment from the PMA1 gene.

A library of Candida albicans genomic DNA was constructed from BamHI-Hind III cut DNA isolated from strain C. albicans WO-1 and inserted into BamHI-HindII cut DNA of pEMBLY-23 as mentioned in Magee et al, Anal. Biochem. 175: 361-372 (1988). The pEMBLY-23 vector was described by Baldari and Cesareni, Gene 35: 27-32 (1985). The library of transformed cells were grown on LB plates supplemented with 50 $\mu$g/ml of ampicillin. Twenty plates were prepared at a density of ~230 colonies per plate or ~4700 colonies total. The colonies were transferred to NEN Colony/Plaque Screen (New England Nuclear). The DNA of the transferred colonies was denatured with 0.5N NaOH and neutralized with 1.0M Tris-HCl pH 7.5 (Grunstein and Hogness (1975) Proc. Natl. Acad. Sci. 72: 3961-3965). Duplicate filters were prepared and hybridized overnight with the ~2kb KpnI Saccharomyces cerevisiae PMA1 fragment. The following morning the filters were washed by standard methods and exposed to XAR-5 film. There were five colonies that contained DNA which hybridized to the 2 kb KpnI probe on duplicate filters. After purification of these five colonies, only one colony, designated 1-10, was reproducibly positive when hybridized to the Saccharomyces 2 kb KpnI fragment. When the DNA from 1-10 was cut with with restriction endonucleases EcoRV, EcoRI, and HindIII and compared to the fragments obtained from chromosomal DNA, it was noted that a portion of the homologous region was missing. This might be expected because the library was prepared from HindIII-BamHI-cut DNA. The 7.0 kb HindIII fragment, the 1.9kb EcoRV fragment, and the 3.4 kb EcoRI fragment are not in clone 1-10. The 0.7 kb EcoRV/EcoRI fragment observed with the chromosomal Candida DNA was seen in the Candida clone 1-10.

A library enriched for the Candida plasma membrane H$^+$ATPase gene was then constructed based on the observation that the Candida and Saccharomyces probes hybridize to a single ClaI fragment of about 12 kb. A portion of C. albicans ATCC 10261 chromosomal DNA was completely digested with ClaI. The digested DNA fragments were separated by gel electrophoresis. A DNA fraction enriched for 10-15 kb fragments was obtained by electroelution of the appropriate segment of electrophoretically separated digested DNA. The collected fragments were ligated into the YEp24 vector DNA. The YEp24 DNA is commercially available from New England Biolabs and has been described by Botstein et al., Gene 8: 17-24 ( 1979). Before ligation, the YEp24 DNA was linearized by complete digestion with ClaI and treated with calf intestine alkaline phosphatase. The ligation products were transformed into Escherichia coli K-12 strain DH5$\alpha$. Library efficiency DH5$\alpha$ cells were obtained from Bethesda Research Laboratories. The transformants were recovered as colonies able to grow on LB plates with 50 $\mu$g/ml of ampicillin. The transformants were collected and plated on ampicillin plates to determine the number of colonies/ml. After the cell titer of the ClaI-YEp24 library was determined, twenty LB plates supplemented with ampicillin were plated with a colony density of 1500 to 2000 library transformed colonies per plate or ~35,000 colonies total. The colonies were transferred to Schleicher & Schuell nitrocellulose filters. The transferred colonies were treated with 0.5N NaOH to denature their DNA and neutralized with 1.0M Tris-HCl pH 7.5 (Grunstein and Hogness Proc. Natl. Acad. Sci. 72:3961-3965 [1975]). Duplicate filters were prepared and hybridized overnight with the 0.7 kb EcoRV/EcoRI fragment from the Candida clone 1-10. The following morning the filters were washed by standard methods and exposed to XAR-5 film. Thirteen colonies with DNA that hybridized to the Candida 0.7 kb EcoRV/EcoRI fragment were recovered and six still hybridized to the 0.7 kb EcoRV/EcoRI fragment when purified. All six putative colonies had the same restriction enzyme pattern and had all the bands that were expected from the Candida chromosomal data. Figure 1 presents the restriction endonuclease map predicted from the mapping data.

EXAMPLE 2

Sequencing the Candida albicans Plasma Membrane ATPase DNA

Since the Candida albicans plasma membrane ATPase (PMA1) gene was cloned by homology to the Saccharomyces cerevisiae PMA1 gene, their DNA sequences should have a high degree of homology to each other and the Saccharomyces sequence should act as a guide for sequencing the Candida DNA. Since the area of highest homology in the Candida clone is the 2.0 kb EcoRV fragment, it was sequenced first. The fragment was subcloned into the EcoRV site of pGEM5zf(+) which is a plasmid commercially available from Promega. A double stranded sequencing procedure was implemented (Lim and Pene (1988) Gene Anal Techn 5: 32-39 and Sequenase kit protocol manufactured by US Biochemical Corp.). T7 and SP6 primers are adjacent upstream and downstream, respectively, to the 2.0 kb EcoRV Candida fragment. The SP6 and T7 are commercially available primers (Promega) and were used to generate approximately 250 bases of sequence into the 2.0 kb fragment. Regions not accessible with the primers were sequenced by use of synthetic oligonucleotides primers.

The DNA sequence of the cloned Candida albicans plasma membrane ATPase is presented in Figure 2. The overall length of the cloned C. albicans DNA was determined to be 2691 base pairs. The cleavage sites for selected restriction endonucleases are indicated above the sequence. The predicted 896 amino acids for the plasma membrane gene are shown below the DNA sequence in Figure 2.

**Claims**

1.   A purified and isolated DNA sequence consisting of essentially a DNA sequence encoding Candida albicans plasma membrane H$^+$ATPase with said sequence free of other Candida albicans DNA.

2.   A purified and isolated DNA sequence comprising the following nucleotide sequence which encodes Candida albicans plasma membrane H$^+$ATPase:

```
TCTATCATTTGTTAATATTTATTTATACCAAGCACCATATAAATACCTAGTTTTTTTTTT
---------+---------+---------+---------+---------+---------+  60

TTTTTTTGTTTGTTAAATCACTTTTTTTTTTCAATCTTTGTTTTTGGTTAATTAATCTTAA
---------+---------+---------+---------+---------+---------+ 120

GAATAAGGGATTTTTATATATATATAAACCATGAGTGCTACTGAACCAACCAACGAAAAG
---------+---------+---------+---------+---------+---------+ 180

GTTGATAAAATCGTCTCCGATGATGAAGACGAAGACATTGACCAATTAGTCGCTGATTTA
---------+---------+---------+---------+---------+---------+ 240

CAATCTAACCCAGGTGCTGGTGATGAAGAAGAAGAGGAGGAAAATGACTCTTCCTTCAAA
---------+---------+---------+---------+---------+---------+ 300

GCCGTCCCAGAAGAATTATTGCAAACTGACCCAAGAGTTGGTTTGACTGATGATGAAGTC
---------+---------+---------+---------+---------+---------+ 360

ACCAAAAGAAGAAAGAGATACGGTTTGAATCAAATGGCTGAAGAACAAGAAAACTTGGTT
---------+---------+---------+---------+---------+---------+ 420

CTTAAATTCGTCATGTTCTTTGTTGGTCCAATTCAATTCGTTATGGAAGCCGCTGCTGTT
---------+---------+---------+---------+---------+---------+ 480
```

8

```
TTGGCTGCTGGTTTAGAAGATTGGGTCGATTTCGGTGTTATCTGTGCTTTATTGTTATTG
---------+---------+---------+---------+---------+---------+ 540


AATGCTTTTGTTGGTTTTATCCAAGAATACCAAGCTGGTTCTATTGTCGATGAATTGAAA
---------+---------+---------+---------+---------+---------+ 600


AAGACTTTGGCCAACTCTGCTCTTGTTGTTAGAAACGGTCAATTAGTTGAAATCCCAGCT
---------+---------+---------+---------+---------+---------+ 660


AACGAAGTTGTTCCAGGTGATATCTTGCAATTGGAAGACGGTACCGTTATTCCAACTGAT
---------+---------+---------+---------+---------+---------+ 720


GGTAGAATTGTTTCTGAAGATTGTTTGTTACAAGTTGATCAATCTGCTATTACTGGTGAA
---------+---------+---------+---------+---------+---------+ 780


TCTTTAGCTGTCGACAAAAGAAGTGGTGACTCTTGTTACTCTTCTTCTACTGTTAAGACT
---------+---------+---------+---------+---------+---------+ 840


GGTGAAGCCTTTATGATTGTTACTGCTACTGGTGACTCTACTTTCGTCGGTAGAGCTGCT
---------+---------+---------+---------+---------+---------+ 900


GCTTTGGTTAACAAAGCTTCCGCTGGTACTGGTCATTTCACTGAAGTCTTGAACGGTATT
---------+---------+---------+---------+---------+---------+ 960


GGTACTACCTTGTTGGTCTTTGTCATTGTTACTTTGTTGGTCGTTTGGGTTGCTTGTTTC
---------+---------+---------+---------+---------+---------+1020


TACAGAACCGTTAGAATTGTTCCAATCTTGAGATACACTTTAGCCATCACTATTATTGGT
---------+---------+---------+---------+---------+---------+1080


GTTCCAGTTGGTTTGCCAGCTGTCGTTACCACTACCATGGCTGTCGGTGCTGCTTACTTG
---------+---------+---------+---------+---------+---------+1140


GCCAAGAAACAAGCTATTGTCCAAAAATTGTCTGCCATTGAATCTTTGGCTGGTGTTGAA
---------+---------+---------+---------+---------+---------+1200
```

```
ATCTTGTGTTCCGATAAAACCGGTACTTTGACCAAGAACAAATTGTCCTTGCACGAACCA
---------+---------+---------+---------+---------+---------+1260

TACACTGTTGAAGGTGTTGAACCAGATGACTTGATGTTGACTGCTTGTTTAGCTGCTTCT
---------+---------+---------+---------+---------+---------+1320

AGAAAGAAGAAGGGTTTGGATGCCATTGATAAAGCTTTCTTGAAATCTTTGATCAACTAC
---------+---------+---------+---------+---------+---------+1380

CCAAGAGCTAAAGCTGCTTTGCCAAAATACAAGGTTATTGAATTCCAACCTTTCGATCCT
---------+---------+---------+---------+---------+---------+1440

GTCTCCAAGAAAGTTACTGCTATTGTTGAATCACCAGAAGGTGAAAGAATTATTTGTGTT
---------+---------+---------+---------+---------+---------+1500

AAGGGTGCCCCATTATTCGTCTTAAAGACTGTTGAAGATGACCACCCAATCCCAGAAGAT
---------+---------+---------+---------+---------+---------+1560

GTCCACGAAAACTACCAAAACACCGTTGCCGAATTTGCTTCCAGAGGTTTCAGATCTTTG
---------+---------+---------+---------+---------+---------+1620

GGTGTTGCCAGAAAGAGAGGTGAAGGTCACTGGGAAATTTTGGGTATTATGCCATGTATG
---------+---------+---------+---------+---------+---------+1680

GATCCACCAAGAGATGATACTGCTGCCACAGTCAATGAAGCTAGAAGATTAGGTTTAAGA
---------+---------+---------+---------+---------+---------+1740

GTTAAGATGTTAACTGGTGATGCCGTTGGTATTGCTAAAGAAACTTGTCGTCAATTAGGT
---------+---------+---------+---------+---------+---------+1800

TTGGGTACTAACATTTACGATGCCGACAGATTAGGTTTGTCCGGTGGTGGTGACATGGCT
---------+---------+---------+---------+---------+---------+1860

GGTTCTGAAATTGCTGATTTCGTTGAAAATGCCGATGGTTTCGCTGAAGGTTTTTCCCGAC
---------+---------+---------+---------+---------+---------+1920
```

10

```
AACGATAAATATAATGCCGTTGAAATCTTACAATCTAGAGGTTACTTGGTTGCCATGACT
---------+---------+---------+---------+---------+---------+1980


GGTGATGGTGTTAACGATGCCCCATCTTTGAAGAAAGCTGATACTGGTATTGCCGTCGAA
---------+---------+---------+---------+---------+---------+2040


GGTGCTACTGATGCTGCCCGTTCTGCTGCCGATATTGTTTTCTTGGCCCCAGGTTTGTCT
---------+---------+---------+---------+---------+---------+2100


GCCATTATTGATGCTTTGAAAACTTCTAGACAAATTTTCCACAGAATGTACTCTTATGTT
---------+---------+---------+---------+---------+---------+2160


GTTTACCGTATTGCCCTTTCATTGCACTTGGAATTGTTCTTAGGTTTATGGATTGCTATC
---------+---------+---------+---------+---------+---------+2220


TTGAACAGATCTTTGGATATTAACTTGATTGTCTTCATTGCCATTTTCGCTGATGTTGCC
---------+---------+---------+---------+---------+---------+2280


ACTTTGGCCATTGCTTATGATAATGCTCCATACGATCCAAAACCAGTTAAATGGAACTTG
---------+---------+---------+---------+---------+---------+2340


CCAAGATTGTGGGGTATGTCCATTGTCTTGGGTGTTATTTTGGCTATTGGTACTTGGATT
---------+---------+---------+---------+---------+---------+2400


ACTTTGACTACTATGTTGTTGCCAAAAGGTGGTATTATCCAAAACTTTGGTGGTTTAGAT
---------+---------+---------+---------+---------+---------+2460


GGTATTTTGTTCTTGCAAATTTCCTTGACTGAAAATTGGTTGATTTTCGTCACCAGAGCT
---------+---------+---------+---------+---------+---------+2520


CAAGGTCCATTCTGGTCATCAATCCCATCATGGCAATTATCTGGTGCTGTCTTGATTGTT
---------+---------+---------+---------+---------+---------+2580


GATATCACTGCCACTTGTTTCACCTTGTTTGGTTGGTGGTCTCAAAACTGGACTGACATT
---------+---------+---------+---------+---------+---------+2640
```

```
GTCACTGTTGTCAGAACCTGGATTTGGTCTTTCGGTGTCTTCTGTGTCATGGGTGGTGCT
---------+---------+---------+---------+---------+---------+2700


TACTACTTGATGTCTACTTCCGAAGCCTTTGACAACTTCTGTAACGGTAGAAAACCACAA
---------+---------+---------+---------+---------+---------+2760


CAACACACTGACAAGAGATCCTTGGAAGATTTCCTTGTGTCTATGCAAAGAGTATCTACT
---------+---------+---------+---------+---------+---------+2820


CAACACGAAAAATCTACTTAAATTTGATGATATCGTTGTTGATGTGAATAATTGGAGATT
---------+---------+---------+---------+---------+---------+2880


AATATTAATCGACAATTGTTATTACTAATTTTGCATGTTTGTTTTTGTCATTG
---------+---------+---------+---------+---------+--- 2933
```

and any altered forms that result in the expression of a biologically active <u>Candida albicans</u> plasma membrane H+ATPase.

3. A vector containing the DNA sequence of claim 1.

4. A vector containing the DNA sequence of claim 2.

5. A host cell transformed by the vector of claim 3 containing the DNA sequence coding for <u>Candida albicans</u> plasma membrane H+ATPase.

6. A host cell transformed by the vector of claim 4 containing the DNA sequence coding for <u>Candida albicans</u> plasma membrane H+ATPase.

7. A process for the production of <u>Candida albicans</u> plasma membrane H+ATPase comprising culturing the transformed microbial host of claim 5 under conditions suitable for the expression of <u>Candida albicans</u> Plasma membrane H+ATPase and recovering <u>Candida Albicans</u> plasma membrane H+ATPase.

8. A process for the production of <u>Candida albicans</u> plasma membrane H+ATPase comprising culturing the transformed microbial host of claim 6 under conditions suitable for the expression of <u>Candida albicans</u> plasma membrane H+ATPase and recovering <u>Candida albicans</u> Plasma membrane H+ATPase.

9. A biologically active, homogeneously pure protein comprising an amino acid sequence substantially corresponding to all or a biologically active portion of the amino acid sequence of <u>Candida albicans</u>.

10. A recombinant Candida albicans plasma membrane H+ATPase comprising at least the following amino acid sequence:

```
      1                              10                              20
MetSerAlaThrGluProThrAsnGluLysValAspLysIleValSerAspAspGluAsp

                                    30                              40
GluAspIleAspGlnLeuValAlaAspLeuGlnSerAsnProGlyAlaGlyAspGluGlu

                                    50                              60
GluGluGluGluAsnAspSerSerPheLysAlaValProGluGluLeuLeuGlnThrAsp

                                    70                              80
ProArgValGlyLeuThrAspAspGluValThrLysArgArgLysArgTyrGlyLeuAsn

                                    90                             100
GlnMetAlaGluGluGlnGluAsnLeuValLeuLysPheValMetPhePheValGlyPro

                                   110                             120
IleGlnPheValMetGluAlaAlaAlaValLeuAlaAlaGlyLeuGluAspTrpValAsp
```

```
                        130                              140
    PheGlyValIleCysAlaLeuLeuLeuLeuAsnAlaPheValGlyPheIleGlnGluTyr

                        150                              160
    GlnAlaGlySerIleValAspGluLeuLysLysThrLeuAlaAsnSerAlaLeuValVal

                        170                              180
    ArgAsnGlyGlnLeuValGluIleProAlaAsnGluValValProGlyAspIleLeuGln

                        190                              200
    LeuGluAspGlyThrValIleProThrAspGlyArgIleValSerGluAspCysLeuLeu

                        210                              220
    GlnValAspGlnSerAlaIleThrGlyGluSerLeuAlaValAspLysArgSerGlyAsp

                        230                              240
    SerCysTyrSerSerSerThrValLysThrGlyGluAlaPheMetIleValThrAlaThr

                        250                              260
    GlyAspSerThrPheValGlyArgAlaAlaAlaLeuValAsnLysAlaSerAlaGlyThr

                        270                              280
    GlyHisPheThrGluValLeuAsnGlyIleGlyThrThrLeuLeuValPheValIleVal

                        290                              300
    ThrLeuLeuValValTrpValAlaCysPheTyrArgThrValArgIleValProIleLeu

                        310                              320
    ArgTyrThrLeuAlaIleThrIleIleGlyValProValGlyLeuProAlaValValThr

                        330                              340
    ThrThrMetAlaValGlyAlaAlaTyrLeuAlaLysLysGlnAlaIleValGlnLysLeu

                        350                              360
    SerAlaIleGluSerLeuAlaGlyValGluIleLeuCysSerAspLysThrGlyThrLeu

                        370                              380
    ThrLysAsnLysLeuSerLeuHisGluProTyrThrValGluGlyValGluProAspAsp

                        390                              400
    LeuMetLeuThrAlaCysLeuAlaAlaSerArgLysLysLysGlyLeuAspAlaIleAsp

                        410                              420
    LysAlaPheLeuLysSerLeuIleAsnTyrProArgAlaLysAlaAlaLeuProLysTyr

                        430                              440
    LysValIleGluPheGlnProPheAspProValSerLysLysValThrAlaIleValGlu
```

                                    450                                    460
          SerProGluGlyGluArgIleIleCysValLysGlyAlaProLeuPheValLeuLysThr

                                    470                                    480
          ValGluAspAspHisProIleProGluAspValHisGluAsnTyrGlnAsnThrValAla

                                    490                                    500
          GluPheAlaSerArgGlyPheArgSerLeuGlyValAlaArgLysArgGlyGluGlyHis

                                    510                                    520
          TrpGluIleLeuGlyIleMetProCysMetAspProProArgAspAspThrAlaAlaThr

                                    530                                    540
          ValAsnGluAlaArgArgLeuGlyLeuArgValLysMetLeuThrGlyAspAlaValGly

                                    550                                    560
          IleAlaLysGluThrCysArgGlnLeuGlyLeuGlyThrAsnIleTyrAspAlaAspArg

                                    570                                    580
          LeuGlyLeuSerGlyGlyGlyAspMetAlaGlySerGluIleAlaAspPheValGluAsn

                                    590                                    600
          AlaAspGlyPheAlaGluGlyPheProAspAsnAspLysTyrAsnAlaValGluIleLeu

                                    610                                    620
          GlnSerArgGlyTyrLeuValAlaMetThrGlyAspGlyValAsnAspAlaProSerLeu

                                    630                                    640
          LysLysAlaAspThrGlyIleAlaValGluGlyAlaThrAspAlaAlaArgSerAlaAla

                                    650                                    660
          AspIleValPheLeuAlaProGlyLeuSerAlaIleIleAspAlaLeuLysThrSerArg

                                    670                                    680
          GlnIlePheHisArgMetTyrSerTyrValValTyrArgIleAlaLeuSerLeuHisLeu

                                    690                                    700
          GluLeuPheLeuGlyLeuTrpIleAlaIleLeuAsnArgSerLeuAspIleAsnLeuIle

                                    710                                    720
          ValPheIleAlaIlePheAlaAspValAlaThrLeuAlaIleAlaTyrAspAsnAlaPro

                                    730                                    740
          TyrAspProLysProValLysTrpAsnLeuProArgLeuTrpGlyMetSerIleValLeu

                                    750                                    760
          GlyValIleLeuAlaIleGlyThrTrpIleThrLeuThrThrMetLeuLeuProLysGly

```
                    770                              780
GlyIleIleGlnAsnPheGlyGlyLeuAspGlyIleLeuPheLeuGlnIleSerLeuThr

                    790                              800
GluAsnTrpLeuIlePheValThrArgAlaGlnGlyProPheTrpSerSerIleProSer

                    810                              820
TrpGlnLeuSerGlyAlaValLeuIleValAspIleThrAlaThrCysPheThrLeuPhe

                    830                              840
GlyTrpTrpSerGlnAsnTrpThrAspIleValThrValValArgThrTrpIleTrpSer

                    850                              860
PheGlyValPheCysValMetGlyGlyAlaTyrTyrLeuMetSerThrSerGluAlaPhe

                    870                              880
AspAsnPheCysAsnGlyArgLysProGlnGlnHisThrAspLysArgSerLeuGluAsp

                    890
PheLeuValSerMetGlnArgValSerThrGlnHisGluLysSerThr
```

and biologically active forms thereof.

FIG. 1

# FIG. 2A

```
TCTATCATTTGTTAATATTTATTTATACCAAGCACCATATAAATACCTAGTTTTTTTTTT
1 ---------+---------+---------+---------+---------+---------+ 60




                                                          A
                                                          f
                                                          l
                                                          I
                                                          I
TTTTTTTGTTTGTTAAATCACTTTTTTTTTTCAATCTTTGTTTTTGGTTAATTAATCTTAA
61 ---------+---------+---------+---------+---------+---------+120


GAATAAGGGATTTTTATATATATATAAACCATGAGTGCTACTGAACCAACCAACGAAAAG
121 ---------+---------+---------+---------+---------+---------+180
                                        MetSerAlaThrGluProThrAsnGluLys



GTTGATAAAATCGTCTCCGATGATGAAGACGAAGACATTGACCAATTAGTCGCTGATTTA
181 ---------+---------+---------+---------+---------+---------+ 240
    ValAspLysIleValSerAspAspGluAspGluAspIleAspGlnLeuValAlaAspLeu



CAATCTAACCCAGGTGCTGGTGATGAAGAAGAAGAGGAGGAAAATGACTCTTCCTTCAAA
241 ---------+---------+---------+---------+---------+---------+ 300
    GlnSerAsnProGlyAlaGlyAspGluGluGluGluGluGluAsnAspSerSerPheLys



GCCGTCCCAGAAGAATTATTGCAAACTGACCCAAGAGTTGGTTTGACTGATGATGAAGTC
301 ---------+---------+---------+---------+---------+---------+ 360
    AlaValProGluGluLeuLeuGlnThrAspProArgValGlyLeuThrAspAspGluVal



ACCAAAAGAAGAAAGAGATACGGTTTGAATCAAATGGCTGAAGAACAAGAAAACTTGGTT
361 ---------+---------+---------+---------+---------+---------+ 420
    ThrLysArgArgLysArgTyrGlyLeuAsnGlnMetAlaGluGluGlnGluAsnLeuVal



CTTAAATTCGTCATGTTCTTTGTTGGTCCAATTCAATTCGTTATGGAAGCCGCTGCTGTT
421 ---------+---------+---------+---------+---------+---------+ 480
    LeuLysPheValMetPhePheValGlyProIleGlnPheValMetGluAlaAlaAlaVal



TTGGCTGCTGGTTTAGAAGATTGGGTCGATTTCGGTGTTATCTGTGCTTTATTGTTATTG
481 ---------+---------+---------+---------+---------+---------+ 540
    LeuAlaAlaGlyLeuGluAspTrpValAspPheGlyValIleCysAlaLeuLeuLeuLeu
```

# FIG. 2B

```
     AATGCTTTTGTTGGTTTTATCCAAGAATACCAAGCTGGTTCTATTGTCGATGAATTGAAA
541  ---------+---------+---------+---------+---------+---------+ 600
     AsnAlaPheValGlyPheIleGlnGluTyrGlnAlaGlySerIleValAspGluLeuLys


                   B
                   a
                   l
                   I
     AAGACTTTGGCCAACTCTGCTCTTGTTGTTAGAAACGGTCAATTAGTTGAAATCCCAGCT
601  ---------+---------+---------+---------+---------+---------+ 660
     LysThrLeuAlaAsnSerAlaLeuValValArgAsnGlyGlnLeuValGluIleProAla


                        E
                        c                        K
                        o                        p
                        R                        n
                        V                        I
     AACGAAGTTGTTCCAGGTGATATCTTGCAATTGGAAGACGGTACCGTTATTCCAACTGAT
661  ---------+---------+---------+---------+---------+---------+ 720
     AsnGluValValProGlyAspIleLeuGlnLeuGluAspGlyThrValIleProThrAsp


                             B
                             c
                             l
                             I
     GGTAGAATTGTTTCTGAAGATTGTTTGTTACAAGTTGATCAATCTGCTATTACTGGTGAA
721  ---------+---------+---------+---------+---------+---------+ 780
     GlyArgIleValSerGluAspCysLeuLeuGlnValAspGlnSerAlaIleThrGlyGlu


                   H
                   i
               S   n
               a   d
               l   I
               I   I
     TCTTTAGCTGTCGACAAAAGAAGTGGTGACTCTTGTTACTCTTCTTCTACTGTTAAGACT
781  ---------+---------+---------+---------+---------+---------+ 840
     SerLeuAlaValAspLysArgSerGlyAspSerCysTyrSerSerSerThrValLysThr


     GGTGAAGCCTTTATGATTGTTACTGCTACTGGTGACTCTACTTTCGTCGGTAGAGCTGCT
841  ---------+---------+---------+---------+---------+---------+ 900
     GlyGluAlaPheMetIleValThrAlaThrGlyAspSerThrPheValGlyArgAlaAla
```

## FIG. 2C

```
                 H
                 i
                 nH
                 dp
                 Ia
                 II
                 /
     GCTTTGGTTAACAAAGCTTCCGCTGGTACTGGTCATTTCACTGAAGTCTTGAACGGTATT
901  ---------+---------+---------+---------+---------+---------+ 960
     AlaLeuValAsnLysAlaSerAlaGlyThrGlyHisPheThrGluValLeuAsnGlyIle


     GGTACTACCTTGTTGGTCTTTGTCATTGTTACTTTGTTGGTCGTTTGGGTTGCTTGTTTC
961  ---------+---------+---------+---------+---------+---------+ 1020
     GlyThrThrLeuLeuValPheValIleValThrLeuLeuValValTrpValAlaCysPhe


     TACAGAACCGTTAGAATTGTTCCAATCTTGAGATACACTTTAGCCATCACTATTATTGGT
1021 ---------+---------+---------+---------+---------+---------+ 1080
     TyrArgThrValArgIleValProIleLeuArgTyrThrLeuAlaIleThrIleIleGly

                 P                         N
                 v                         c
                 u                         o
                 I                         I
                 I
     GTTCCAGTTGGTTTGCCAGCTGTCGTTACCACTACCATGGCTGTCGGTGCTGCTTACTTG
1081 ---------+---------+---------+---------+---------+---------+ 1140
     ValProValGlyLeuProAlaValValThrThrThrMetAlaValGlyAlaAlaTyrLeu


     B
     a
     l
     I
     GCCAAGAAACAAGCTATTGTCCAAAAATTGTCTGCCATTGAATCTTTGGCTGGTGTTGAA
1141 ---------+---------+---------+---------+---------+---------+ 1200
     AlaLysLysGlnAlaIleValGlnLysLeuSerAlaIleGluSerLeuAlaGlyValGlu


     ATCTTGTGTTCCGATAAAACCGGTACTTTGACCAAGAACAAATTGTCCTTGCACGAACCA
1201 ---------+---------+---------+---------+---------+---------+ 1260
     IleLeuCysSerAspLysThrGlyThrLeuThrLysAsnLysLeuSerLeuHisGluPro
```

## FIG. 2D

```
                                H                         X
                                i                         b
                                n                         a
                                d                         I
                                I
     TACACTGTTGAAGGTGTTGAACCAGATGACTTGATGTTGACTGCTTGTTTAGCTGCTTCT
1261 ---------+---------+---------+---------+---------+---------+ 1320
     TyrThrValGluGlyValGluProAspAspLeuMetLeuThrAlaCysLeuAlaAlaSer


                                          B
                                          c
                                          l
                                          I
     AGAAAGAAGAAGGGTTTGGATGCCATTGATAAAGCTTTCTTGAAATCTTTGATCAACTAC
1321 ---------+---------+---------+---------+---------+---------+ 1380
     ArgLysLysLysGlyLeuAspAlaIleAspLysAlaPheLeuLysSerLeuIleAsnTyr


                                     E
                                     c
                                     o
                                     R
                                     I
     CCAAGAGCTAAAGCTGCTTTGCCAAAATACAAGGTTATTGAATTCCAACCTTTCGATCCT
1381 ---------+---------+---------+---------+---------+---------+ 1440
     ProArgAlaLysAlaAlaLeuProLysTyrLysValIleGluPheGlnProPheAspPro


     GTCTCCAAGAAAGTTACTGCTATTGTTGAATCACCAGAAGGTGAAAGAATTATTTGTGTT
1441 ---------+---------+---------+---------+---------+---------+ 1500
     ValSerLysLysValThrAlaIleValGluSerProGluGlyGluArgIleIleCysVal


     AAGGGTGCCCCATTATTCGTCTTAAAGACTGTTGAAGATGACCACCCAATCCCAGAAGAT
1501 ---------+---------+---------+---------+---------+---------+ 1560
     LysGlyAlaProLeuPheValLeuLysThrValGluAspAspHisProIleProGluAsp


                                          B
                                          g
                                          l
                                          I
                                          I
     GTCCACGAAAACTACCAAAACACCGTTGCCGAATTTGCTTCCAGAGGTTTCAGATCTTTG
1561 ---------+---------+---------+---------+---------+---------+ 1620
     ValHisGluAsnTyrGlnAsnThrValAlaGluPheAlaSerArgGlyPheArgSerLeu
```

# FIG. 2E

```
                                                               B
                                                               a
                                                               m
                                                               H
                                                               I
     GGTGTTGCCAGAAAGAGAGGTGAAGGTCACTGGGAAATTTTGGGTATTATGCCATGTATG
1621 ---------+---------+---------+---------+---------+---------+ 1680
     GlyValAlaArgLysArgGlyGluGlyHisTrpGluIleLeuGlyIleMetProCysMet


     GATCCACCAAGAGATGATACTGCTGCCACAGTCAATGAAGCTAGAAGATTAGGTTTAAGA
1681 ---------+---------+---------+---------+---------+---------+ 1740
     AspProProArgAspAspThrAlaAlaThrValAsnGluAlaArgArgLeuGlyLeuArg


            H
           ·i
            nH
            dp
            Ia
            II
             /
     GTTAAGATGTTAACTGGTGATGCCGTTGGTATTGCTAAAGAAACTTGTCGTCAATTAGGT
1741 ---------+---------+---------+---------+---------+---------+ 1800
     ValLysMetLeuThrGlyAspAlaValGlyIleAlaLysGluThrCysArgGlnLeuGly


     TTGGGTACTAACATTTACGATGCCGACAGATTAGGTTTGTCCGGTGGTGGTGACATGGCT
1801 ---------+---------+---------+---------+---------+---------+ 1860
     LeuGlyThrAsnIleTyrAspAlaAspArgLeuGlyLeuSerGlyGlyGlyAspMetAla


     GGTTCTGAAATTGCTGATTTCGTTGAAAATGCCGATGGTTTCGCTGAAGGTTTTCCCGAC
1861 ---------+---------+---------+---------+---------+---------+ 1920
     GlySerGluIleAlaAspPheValGluAsnAlaAspGlyPheAlaGluGlyPheProAsp


                              X
                              b
                              a
                              I
     AACGATAAATATAATGCCGTTGAAATCTTACAATCTAGAGGTTACTTGGTTGCCATGACT
1921 ---------+---------+---------+---------+---------+---------+ 1980
     AsnAspLysTyrAsnAlaValGluIleLeuGlnSerArgGlyTyrLeuValAlaMetThr
```

# FIG. 2F

```
                    H
                    i
                    nH
                    dp
                    Ia
                    II
                     /
      GGTGATGGTGTTAACGATGCCCCATCTTTGAAGAAAGCTGATACTGGTATTGCCGTCGAA
1981  ---------+---------+---------+---------+---------+---------+ 2040
      GlyAspGlyValAsnAspAlaProSerLeuLysLysAlaAspThrGlyIleAlaValGlu


      GGTGCTACTGATGCTGcCCGTTCTGCTGCCGATATTGTTTTCTTGGCCCCAGGTTTGTCT
2041  ---------+---------+---------+---------+---------+---------+ 2100
      GlyAlaThrAspAlaAlaArgSerAlaAlaAspIleValPheLeuAlaProGlyLeuSer


                              X
                              b
                              a
                              I
      GCCATTATTGATGCTTTGAAAACTTCTAGACAAATTTTCCACAGAATGTACTCTTATGTT
2101  ---------+---------+---------+---------+---------+---------+ 2160
      AlaIleIleAspAlaLeuLysThrSerArgGlnIlePheHisArgMetTyrSerTyrVal


      GTTTACCGTATTGCCCTTTCATTGCACTTGGAATTGTTCTTAGGTTTATGGATTGCTATC
2161  ---------+---------+---------+---------+---------+---------+ 2220
      ValTyrArgIleAlaLeuSerLeuHisLeuGluLeuPheLeuGlyLeuTrpIleAlaIle


                    B
                    g
                    l
                    I
                    I
      TTGAACAGATCTTTGGATATTAACTTGATTGTCTTCATTGCCATTTTCGCTGATGTTGCC
2221  ---------+---------+---------+---------+---------+---------+ 2280
      LeuAsnArgSerLeuAspIleAsnLeuIleValPheIleAlaIlePheAlaAspValAla


                    B
                    a
                    l
                    I
      ACTTTGGCCATTGCTTATGATAATGCTCCATACGATCCAAAACCAGTTAAATGGAACTTG
2281  ---------+---------+---------+---------+---------+---------+ 2340
      ThrLeuAlaIleAlaTyrAspAsnAlaProTyrAspProLysProValLysTrpAsnLeu
```

## FIG. 2G

```
    CCAAGATTGTGGGGTATGTCCATTGTCTTGGGTGTTATTTTGGCTATTGGTACTTGGATT
2341 ---------+---------+---------+---------+---------+---------+ 2400
    ProArgLeuTrpGlyMetSerIleValLeuGlyValIleLeuAlaIleGlyThrTrpIle


    ACTTTGACTACTATGTTGTTGCCAAAAGGTGGTATTATCCAAAACTTTGGTGGTTTAGAT
2401 ---------+---------+---------+---------+---------+---------+ 2460
    ThrLeuThrThrMetLeuLeuProLysGlyGlyIleIleGlnAsnPheGlyGlyLeuAsp


    GGTATTTTGTTCTTGCAAATTTCCTTGACTGAAAATTGGTTGATTTTCGTCACCAGAGCT
2461 ---------+---------+---------+---------+---------+---------+ 2520
    GlyIleLeuPheLeuGlnIleSerLeuThrGluAsnTrpLeuIlePheValThrArgAla


    CAAGGTCCATTCTGGTCATCAATCCCATCATGGCAATTATCTGGTGCTGTCTTGATTGTT
2521 ---------+---------+---------+---------+---------+---------+ 2580
    GlnGlyProPheTrpSerSerIleProSerTrpGlnLeuSerGlyAlaValLeuIleVal


    E
    c
    o
    R
    V
    GATATCACTGCCACTTGTTTCACCTTGTTTGGTTGGTGGTCTCAAAACTGGACTGACATT
2581 ---------+---------+---------+---------+---------+---------+ 2640
    AspIleThrAlaThrCysPheThrLeuPheGlyTrpTrpSerGlnAsnTrpThrAspIle


    GTCACTGTTGTCAGAACCTGGATTTGGTCTTTCGGTGTCTTCTGTGTCATGGGTGGTGCT
2641 ---------+---------+---------+---------+---------+---------+ 2700
    ValThrValValArgThrTrpIleTrpSerPheGlyValPheCysValMetGlyGlyAla


    TACTACTTGATGTCTACTTCCGAAGCCTTTGACAACTTCTGTAACGGTAGAAAACCACAA
2701 ---------+---------+---------+---------+---------+---------+ 2760
    TyrTyrLeuMetSerThrSerGluAlaPheAspAsnPheCysAsnGlyArgLysProGln


    CAACACACTGACAAGAGATCCTTGGAAGATTTCCTTGTGTCTATGCAAAGAGTATCTACT
2761 ---------+---------+---------+---------+---------+---------+ 2820
    GlnHisThrAspLysArgSerLeuGluAspPheLeuValSerMetGlnArgValSerThr
```

FIG. 2H

```
                                   E
                                   c
                                   o
                                   R
                                   V
     CAACACGAAAAATCTACTTAAATTTGATGATATCGTTGTTGATGTGAATAATTGGAGATT
2821 --------+---------+---------+---------+---------+---------+ 2880
     GlnHisGluLysSerThrEnd
```

```
     AATATTAATCGACAATTGTTATTACTAATTTTGCATGTTTGTTTTTGTCATTG
2881 --------+---------+---------+---------+---------+--- 2933
```

**European Patent Office**

## EUROPEAN SEARCH REPORT

Application Number

EP 91 30 6542

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | EUROPEAN JOURNAL OF BIOCHEMISTRY vol. 154, 1986, BERLIN, GERMANY pages 375 - 381; M.J.HUBBARD, R.SURARIT, P.A. SULLIVAN AND M.G.SHEPHERD: 'The isolation of plasma membrane and chararcterisation of the plasma membrane ATPase from the yeast Candida Albicans' --- | 1,3,5,9 | C12N15/55 C12N9/14 |
| Y,D | JOURNAL OF BIOLOGICAL CHEMISTRY. vol. 263, no. 36, 25 December 1988, BALTIMORE US pages 19480 - 19487; A.SCHESSER, S.ULASZEWSKI, M. GHISLAIN AND A. GOFFEAU: 'A second Transport ATPase gene in Saccharomyces Cerevisiae' ----- | 1,3,5,9 | |
| | | | TECHNICAL FIELDS SEARCHED (Int. Cl.5 ) C12N C12P |

The present search report has been drawn up for all claims

| Place of search | Date of completion of the search | Examiner |
|---|---|---|
| THE HAGUE | 04 NOVEMBER 1991 | VAN PUTTEN A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)